# EUROPÄISCHE PATENTSCHRIFT

(11) **EP 1 032 407 B1**
(45) Veröffentlichungstag und Bekanntmachung des Hinweises auf die Patenterteilung: **10.09.2003**
(21) Anmeldenummer: 98962374.9
(22) Anmeldetag: 20.11.1998
(51) Int. Cl.: A61K 35/54, A61K 38/23

(54) **VERWENDUNG VON PUTAMEN OVI**
USE OF PUTAMEN OVI
UTILISATION DE PUTAMEN OVI

(30) Priorität: 21.11.1997 DE 19751681
(43) Veröffentlichungstag der Anmeldung: 06.09.2000
(73) Patentinhaber: aar Pharma GmbH & Co. KG, 42853 Remscheid (DE)
(72) Erfinder: RUEPP, Michel O., 42853 Remscheid (DE)
(74) Vertreter: Jönsson, Hans-Peter, Dr.Dipl.-Chem.
(86) Internationale Anmeldenummer: EP9807477
(87) Internationale Veröffentlichungsnummer: WO99026641

(56) Entgegenhaltungen:
- WO-A-97/31021
- DE-A- 4 422 613
- DE-A- 19 627 376
- DE-A- 19 639 375
- DE-A- 19 700 788
- DATABASE WPI Derwent Publications Ltd., London, GB; AN 95-091262 XP002099928 & CN 1 079 149 A (LI H), 8. Dezember 1993

## Beschreibung

Gegenstand der Erfindung ist die Verwendung von aufbereitetem Putamen ovi zur Herstellung oral applizierbarer Arzneimittel und lokal applizierbarem Knochenersatz.

Die Eischale ist als Arzneimittel schon seit 1930 in Gebrauch. Zubereitungen aus Eischalen werden auch heute als Mineralstoffe- und Spurenelemente-lieferndes Mittel zur Substitution insbesondere von Calcium verwendet.

Die WO 96/00578 betrifft ein Verfahren zur Herstellung von Putamen ovi, Putamen ovi mit einer definierten Korngröße sowie die Verwendung von Putamen ovi zur Behandlung von Calciummangelzuständen.

Der Stand der Technik zeigt, daß die Eischale in der Form von standardisierten, oral applizierbaren Präparaten zubereitet wird und zur Calcium-Substitution Verwendung findet.

Demgegenüber besteht die Aufgabe der vorliegenden Erfindung in der Verwendung eines besonders aufbereiteten oral applizierbaren Arzneimittels und lokal applizierbaren Knochenersatzes.

Die vorstehend genannte Aufgabe wird erfindungsgemäß gelöst durch die Verwendung von aufbereitetem Putamen ovi (Eischale von Gallus gallus domesticus), insbesondere saccharidhaltige Granulate mikronisierter Eischalen (PO), Eischalenbestandteile der zentralen Palisadenzone (POM), Schalenmatrix mit organisch gebundenen Mineralstoffen (MPM) zur Herstellung von oral applizierbaren Arzneimitteln zur Behandlung oder Prävention von strahlen-, infektions- und chemisch bedingten Organ- und Gewebeschädigungen **sowie** zur zellulären Immunstimulation **dadurch bedingter** immunsupprimierter Zustände.

Die Organ- und Gewebeschädigungen umfassen:
Schädigungen des O-MALT-Systems des Dünndarms, des Knochenmarks (Knochenmarkaplasie),
Knochen- und Knorpel-Genesestörungen,
Krankheiten des Bewegungsapparates,
Dysfunktion, Aplasie oder Hypoplasie des Thymus,
Dysfunktion der Milz und Aplasie oder Hypoplasie infolge medikamenten- oder strahlenbedingter Schädigung der Lymphknoten,
Atrophie, Nekrose der Leber,
Insuffizienz der exokrinen Sekretion von Proteasen, Esterasen,
Carbohydrasen und Nukleasen der Bauchspeicheldrüse,
Insuffizienz der endokrinen Funktion der Langerhansschen Insein, des Kohlenhydratstoffwechsels und der Nieren,
Leukozytopenie, Granulozytopenie, Lymphozytopenie, Thrombozytopenie, Erythrozytopenie,
Immunglobulinmangelzustände, auch infolge von AIDS und Tumoren sowie
Hyperlipoproteinämien und Hyperlipidämie.

Die zelluläre Immunstimulation ist dadurch gekennzeichnet, dass sie insbesondere nach einer Immunsuppression nach Strahlen- oder Chemotherapeutikabehandlung zur Erhöhung der Zellzahlen von Leukozyten, Thrombozyten, Erythrozyten, segmentkernigen Granulozyten und Lymphozyten, T-, B-, Helfer-, Suppressor- und NK-Zellen, insbesondere von B-Lymphozyten in der terminalen Strombahn betrifft, wobei eine Zunahme vitaler Lymphozyten in der Rindenzone und den -follikeln in den Peyerschen Plaques erkennbar ist, die mit einer sekretorischen Induktion von sIGA in den Dünndarm verbunden ist und/oder
eine zelluläre Stimulation immunkompetenter Zellen in Thymus, Milz und mesenterialen Lymphknoten betrifft und/oder
die Aktivierung der Stoffwechselfunktionen von Leber, Pankreas und Nieren durch Senkung der Serumwerte von Kreatinin, Harnsäure, Glukose, Bilirubin, Cholesterin und Triglyzeriden sowie GOT, GPT und GGT betrifft,

Weitere Organ- und Gewebeschadigungen betreffen primäre oder sekundäre Störungen oder Schädigungen chondraler oder desmaler Ossifikation, der Osteoneogenese und Hämatogenese, gegebenenfalls eine Behandlung in Kombination mit Natriumfluorid und Hormonen, insbesondere Östrogenen, Calcitonin, Pyrophosphate, insbesondere Biphosphonate und Vitaminen, insbesondere Vitamin D, insbesondere D3 und Dihydroxicolecalciferol,
der Knochensubstanz mit äußerer Substantia corticalis und innerer Substantia spongiosa,
des Skelettes inklusive Knochenzellen, Interzellulärsubstanz mit kollagenen Fibrillen und verkalkter Kittsubstanz,
des Knochenmetabolismus inklusive der Funktion von Osteoklasten und Osteoblasten und
der Abstimmung zwischen Knochenresorption und -formation.

Die Verwendung von Putamen ovi führt zur Stimulation der Stoffwechselleistung der Osteoblasten-spezifischen Transkriptionsfaktoren, der Differenzierung der Vorläuferzellen zu Osteoblasten durch Regulierung der Osteoblastendifferenzierung und Stimulation der Bereitstellung der knochenspezifischen alkalischen Phosphatase, der kollagene, insbesondere Kollagen Typ I sowie von Osteopontin und Osteocalcin; und
in der Kiefer- und Knochenchirurgie nach oraler und lokaler Applikation zur Stimulierung der Osteoneogenese und der frühzeitigen Kallusbildung.

Ferner umfaßt die Erfindung die Verwendung von Putamen ovi in Kombination mit Zytostatika, insbesondere Cyclophosphamid oder Strahlentherapie als orale Adjuvanzien der malignen Tumorbehandlung, insbesondere der Behandlung von primären und sekundären Knochentumoren mit osteoklastischen oder - lytischen Metastasen, tumorähnlichen Knochendefekten mit intraossärer Raumforderung, zur Behandlung von Knochenzysten; und
zur Steigerung der Verträglichkeit von chemo-, zytostatischer, radiologischer Therapie, insbesondere in Kombination mit Zystostatika, insbesondere Cisplatin, Cyclophosphamid, Methotrexat, Fluorouracil, Bleömycin und/oder Diuretika, insbesondere Acetazolamid, Hydrochlorothiazid, Chlorthalidon, Furosemid, Amilorid zur Hemmung des Diurese- und Salurese-bedingten Knochensubstanzabbaues, als
Adjuvanz der Analgesie und
zur positiven pharmazeutischen Beeinflussung der Wechselwirkungen zwischen Endokrinum, Nerven- und Immunsystem, ferner
zur Hemmung der ossären Entkalkung bei täglichem Zigarettenkonsum und
zur additiven Behandlung von Hyperlipoproteinämien, insbesondere Hypercholesterinämie und/oder Hypertriglyzeridämie; ferner zur Behandlung von Anämie bei Tumorpatienten.

Die Knochenstruktur unterliegt bekanntermaßen einer Vielzahl von Regulationsmechanismen. Es ist allgemein bekannt, daß die Mineralstoffe Calcium, Phosphor und Magnesium ganz wesentlich am Knochenstoffwechsel beteiligt sind. Darüber hinaus sind aber auch eine Reihe bestimmter Spurenelemente wie Mangan, Kupfer und Zink als Cofaktoren für die Kollagen- und Mukopolysaccharid-Synthese für die Aufrechterhaltung des Stoffwechselgleichgewichtes erforderlich.

Der Mangel an derartigen essentiellen Spurenelementen führt zu einer pathophysiologischen Situation mit Störungen enzymatischer Reaktionen in den Zellen des Hartgewebes. Diese nachteiligen Effekte auf den Metabolismus des Knochens können auch konsekutiv auf das Immunsystem übergreifen.

Vergleicht man unter diesen Aspekten humanes mit tierischem Hartgewebe, so läßt sich ein auffallend ähnliches Spektrum der Mineralstoffe und Spurenelemente in Knochen, Zähnen, aber auch im extraossär durch Membranpassage gebildeten Putamen ovi (Eischale, Gallus gallus domesticus) nachweisen. Diese Übereinstimmung trifft im Vergleich von Knochen mit Putamen ovi (PO) nicht nur auf die anorganischen sondern auch auf bestimmte organische Verbindungen wie Mukopolysaccharide auf der Basis von Chondroitinsulfat und Hyaluronsäure zu.

Im Falle der Species Gallus werden während der Embryonalphase aus der PO-Matrixmembran Mineralstoffe und Spurenelemente dem Skelettsystem des sich entwickelnden Organismus bioverfügbar präsentiert; ein Vorgang, der mit der Osteoneogenese des Säugetierorganismus und des Menschen vergleichbar ist.

In den erfindungsgemäßen Putamen ovi-Präparaten sind die für den Knochenstoffwechsel notwendigen biologisch wirksamen organischen und überwiegend komplex gebundenen anorganischen Bestandteile wie Calcium, Eisen, Fluor, Kalium, Kieselsäure, Magnesium, Kupfer, Molybdän, Selen, Zink u. a. weitgehend bioverfügbar für therapeutische Anwendungsmöglichkeiten enthalten.

Da erniedrigte Serumkonzentrationen von Mangan (Mn), Kupfer (Cu) und Zink (Zn) mit schlecht heilenden Knochenfrakturen assoziiert sind, ein Mangandefizit über längere Zeit Osteoporose induziert und die Strontium-Konzentration mit der Knochendichte (g Calcium-Hydroxyapatit/cm³) negativ korreliert, ist Putamen ovi aufgrund seines physiologischen Gehalts an Spuren- und Ultraspurenelementen ein therapeutisch wirksames Substitutionsprodukt für diese Elemente (insbesondere für Mangan, Kupfer, Zink, Strontium, Molybdän, Vanadium, Kobalt, Fluor, Eisen, Magnesium u.a.) sowie ein wirksames Naturstoff-Therapeutikum zur Verbesserung des Knochenmetabolismus inklusive der Funktion von Osteoblasten (Knochengeweberemodellierung).

Bei der Herstellung von PO werden besonders validierte Anforderungen an den Wirkstoff hinsichtlich Auswahl (Qualität, Reinheit), Aufbereitung (Reinheit, Sterilität) sowie Galenik (Standardisierung, Bioverfügbarkeit) eingehalten.

Unter diesen Voraussetzungen wurde in einer Patientenstudie gezeigt, daß es möglich ist, dosisabhängig durch PO-Behandlung die Progression einer Knochendichte-Verminderung zu hemmen und sogar den Effekt umzukehren, d. h. eine Osteoneogenese zu induzieren.

Die Wirkstoffe der Eischalen können erfindungsgemäß wie folgt eingeteilt werden:
1. Eischalenbestandteile der zentralen Palisadenzone, befreit von der inneren und äußeren Schalenmembran (PO)
   Die zentrale Palisadenzone besteht inbesondere aus der Eischale, die von der Schalenmembran oder von der Schalenmembran und der Schalenmatrix durch Proteasen oder alkalische Denaturierung und anschließende Reinigung befreit ist oder durch die Verwendung der Palisadenmatrix, die entweder durch Decalcifikation oder durch wässrige und/oder organische Lösungsmittel, wie auch mittels überkritischer Gase (CO₂) gewonnen wird.
2. Eischalenbestandteile der zentralen Palisadenzone, befreit von der inneren und äußeren Schalenmembran sowie der Schalenmatrix der Palisadenzone (POM)
3. Schalenmatrix der Palisadenzone mit organisch gebundenen Mineralstoffen (MPM).

Eine bevorzugte Ausführungsform der Erfindung betrifft die Verwendung von Putamen ovi zur oralen und lokalen Applikation von PO, POM und MPM als Pulver, Granulat oder Paste als Knochenzement, zur Knochenremodellierung und Implantation sowie als Knochenersatz mit osteoneogenese-aktivem Potential zur gezielten lokalen Knochenbildung. Ferner ist die Kombination mit Viscum-, Echinacea-, Cynara-, Urtica-, Betula-, Equisetum und/oder Taraxacum-Extrakten möglich.

Weitere bevorzugte Ausführungsformen der Erfindung sind den abhängigen Patentansprüchen zu entnehmen.

In den Untersuchungen zur vorliegenden Erfindung wurden in einer Studie an Ratten(Behandlungsdauer7Tage) mitPutamenovimikronisiert(PO) in der galenischen Zubereitung als Drageekemgranulat gemäß WO 96/00578 definierte Funktionen von Knochen, Knochenmark, des Kreislauf- und Immunsystems sowie von Leber, Pankreas und Nieren beeinflußt, auch unter der simultanen Applikation mit Cyclophosphamid (CyS) und Furosemid (FUR).

Der Einfluß von PO auf klinisch gesunde Ratten (Gruppe II) kann wie folgt zusammengefaßt werden:

Die verwendete Prüfsubstanz PO
- hatte keinen Einfluß auf die durchschnittliche Zunahme des Körpergewichts sowie der Organgewichte von Milz und Thymus der Versuchstiere,
- induzierte im peripheren Blut
   eine geringgradige Zunahme der Zellzahlen von segmentkernigen Granulozyten, B- und Suppressor-Zellen sowie eine geringgradige Abnahme der Helfer-Zellen,
- führte im Knochenmark
   zu einer gesteigerten Tendenz der Bildung von zellreichen Nestern der Granulo-, Erythro-, Lympho- und Thrombozytopoese mit funktioneller Kernschwellung und darüberhinaus zu aktivierten Osteozyten (keine Ruhekeme) und aktivierten Osteoblasten, angelagert an den Trabekeln der Spongiosa,
- bewirkte im Thymus
   eine Zunahme lymphoblastischer Zellelemente in Cortex und Medulla, dabei war auffällig, daß aufgrund der Zellzahlzunahme lichtoptisch eine entsprechende Zonengliederung in Cortex und Medulla aufgehoben war,
- führte in der Milz
   zu einer nur geringgradigen Verbreiterung der Marginalzonen insbesondere im Bereich der periarteriellen Scheiden (PALS),
- induzierte im mesenterialen Lymphknoten
   eine beträchtliche Zunahme lymphozytärer Zellelemente in den B- und T-Lymphozytenarealen sowie in den Marksträngen, so daß auch hier die entsprechenden Areale lichtoptisch nicht differenziert erschienen,
- stimulierte deutlich die Leistung des Leberstoffwechsels
   morphologisch in Form einer funktionellen Zellkernschwellung mit dem Bild einheitlich großer Zellkerne (Gleichschaltung)
- bewirkte im peripheren Blut
   eine Abnahme der Gehaltswerte von Kreatinin, Bilirubin, Harnstoff, GLDH, AP, SP, Cholesterin und Triglyzeriden; der Calciumspiegel blieb unbeeinflußt.

Die simultane Applikation von CyS und PO (Gruppe VII)
- zeigte eine prozentuale Abnahme der durchschnittlichen Körpergewichtsentwicklung sowie der Organgewichte von Milz und Thymus der Versuchstiere
- induzierte im peripheren Blut
   a) im Vergleich mit den Kontrollwerten (Gruppe I)
      eine Abnahme der Leukozyten (- 37, 4 %) und ihrer Subsets (z. B. T-Lymphozyten: - 20, 7 %), die wertmäßig geringer ausfiel als diejenige der Gruppe V (CyS: WBC - 51, 3 %, z. B. T-Lymphozyten - 51 %) und der Gruppe VIII (CyS + CC: WBC - 51, 8 %, z. B. T-Lymphozyten - 46,5%) sowie der Gruppe IX (CyS + NF: WBC - 39 %, z. B. T-Lympho-zyten - 23, 4 %),
   b) im Vergleich mit CyS (Gruppe V)
      eine Zunahme der Zellzahlen von Leukozyten (+ 28, 4 %), segmentkemigen Granulozyten (+ 4, 3 %) sowie der Lymphozyten (+ 35 %), T- (+ 64, 8 %), B-(+ 69, 2 %), Helfer- (+ 44, 4 %) und Suppressor-Zellen (+ 23, 2 %) als Ausdruck eines zellulären chemo-protektiven Effektes,
   c) im Vergleich mit CyS + Calciumcarbonat (CC) (Gruppe VIII) eine Zunahme der Zellzahlen, die mit derjenigen korreliert, die mit der Gruppe V (CyS) ermittelt wurde, d. h. ein chemo-protektiver Effekt ist bei reinem Calciumcarbonat nicht vorhanden,
   d) im Vergleich mit CyS + Natriumfluorid (NF) (Gruppe IX) eine Zunahme der Zellzahlen, die geringer ausfällt als bei CyS + PO (Gruppe VII)
- führte im Knochenmark
   im Vergleich mit CyS (Gruppe V)
   zu einer deutlich gesteigerten Tendenz der Bildung von zellreichen Nestern (Environments) der Granulo-, Erythro-, Lympho- und Thrombozytopoese (vergleichbar mit dem Zellbild der Kontrolle): keine auffällige Zellzahlverminderung, keine Anzeichen einer Degeneration kernhaltiger Zellen, unveränderte Erythrozyten in den Sinus, d. h. Erythrozyten mit Stechapfelform (Akanthozyten), wie sie unter dem Einfluß von CyS auch in Kombination mit CC und NF auftreten, sind nicht vorhanden.
   Auch in der Gruppe VII (CyS + PO) - im Vergleich mit Gruppe II (PO) - werden aktivierte Osteozyten und Osteoblasten sowie eine aktive Zellteilung angetroffen,
- bewirkte im Thymus
   im Vergleich mit CyS (Gruppe V), CyS + CC (Gruppe VIII) und CyS + NF (Gruppe IX)
   eine deutliche Zunahme lymphoblastischer Zellelemente in Kortex und Medulla
- führte in der Milz
   im Vergleich mit CyS (Gruppe V), CyS + CC (Gruppe VIII) und CyS + NF (Gruppe IX)
   zu einer Verbreiterung der Marginalzonen insbesondere im Bereich der periarteriellen Scheiden (PALS)
- stimulierte in den Leberzellen
   im Vergleich mit CyS (Gruppe V)
   die Stoffwechselleistung, d. h. partielle funktionelle Zellkernschwellung in den Hepatozyten,
- induzierte im mesenterialen Lymphknoten
   im Vergleich mit CyS (Gruppe V), CyS + CC (Gruppe VIII) und CyS + NF (Gruppe IX)
   eine beträchtliche Zunahme lymphozytärer Zellelemente insbesondere in den T-Lymphozytenarealen sowie in den Marksträngen,
- bewirkte im peripheren Blut im Vergleich mit CyS (Gruppe V)
   eine Zunahme von Kalium (+ 15, 6 %) und saurer Phosphatase (+ 5, 8 %), so daß letztere Parameter das Gehaltsniveau im Serum der Kontrollgruppe erreichte.

Calciumcarbonat (CC) zeigte weder bei klinisch gesunden Ratten positive Effekte noch nach simultaner Cyclophosphamid-Applikation chemo-protektive Wirkungen.

Die vorliegende Erfindung bestätigt die klinisch erhobenen Befunde aus einer 2-Jahres-Studie mit 361 gesunden Frauen in der Postmenopause, bei denen die Wirkung von Calciumcarbonat und einer organischen Calciumverbindung auf die osteopenische Progression der Knochendichteminderung geprüft wurde. Dabei zeigte die Behandlung mit reinem Calciumcarbonat im Gegensatz zur "organischen" Calciumverbindung lediglich einen geringgradig ausgeprägten hemmenden Einfluß auf den Knochenmasseverlust.

Natriumfluorid (NF) induzierte keinen Effekt hinsichtlich der Zellzahlvermehrung von T-, Helfer- oder Suppressor-Zellen in der terminalen Strombahn; im Gegensatz hierzu wurde jedoch die Anzahl der B-Lymphozyten und segmentkemigen Granulozyten erhöht (selektive Induktion).

Ein chemo-protektiver Effekt zeigte sich bei NF nach simultaner Applikation mit CyS bei Leukozyten im peripheren Blut, nicht jedoch im Knochenmark.

Die Anzahl der Erythrozyten wurde beim gesunden Tier durch CC nicht, durch PO leicht und durch CyS deutlich erhöht, hingegen durch NF gesenkt.

Nach der simultanen Applikation von CyS und PO wurde die Erythrozytenzahl im Vergleich mit den erhöhten Werten von CyS normalisiert, d. h. gesenkt (substanzspezifischer Ausschüttungseffekt von CyS wurde gehemmt).

Der senkende Effekt auf die Erythrozytenzahl durch NF und der Ausschüttungseffekt von CyS wurden nach simultaner Applikation kompensiert; in diesem Fall korrelierte der Wert mit dem der Kontrollgruppe, nicht jedoch in Bezug auf das Knochenmark.

Als therapeutisch wünschenswert bei der Applikation von Putamen ovi werden angesehen:
- die generelle Zunahme definierter immunkompetenter Zellen im Knochenmark, in primären und sekundären lymphatischen Organen und in der terminalen Strombahn - insbesondere unter dem simultanen Einfluß zytotoxischer Substanzen (Zytostatika, Kortikoide, Diuretika u. a.) als Ausdruck eines chemo-protektiven (-präventiven) Effektes). Dies konnte auch klinisch durch eine Hemmung des Rückganges immunkompetenter Zellen sowie die Demineralisierung des Hartgewebes gesichert werden.
- die gesteigerte Tendenz der Granulo-, Erythro-, Lympho- und Thrombozytopoese wie auch die Aktivierung der Osteoblasten und Osteozyten als Zeichen einer gesteigerten Hämato-, Angio- und Osteogenese. Dies konnte auch klinisch indirekt durch die Knochendichtezunahme und gleichzeitige Reduktion des Belastungsschmerzes des Bewegungsapparates bestätigt werden.
- die Stimulation der Stoffwechselleistung der Hepatozyten als funktionelles Äquivalent für eine hepatokurative und -protektive Wirkung,
- die Abnahme der Serumwerte von Bilirubin, Kreatinin und Harnstoff als Zeichen einer Verbesserung der renalen Funktion und somit der Eritgiftung des im Eiweißstoffwechsel entstehenden Ammoniaks und im Zusammenhang mit diesem Reaktionszyklus eine gesteigerte mitochondriale Leistung der Hepatozyten,
- die Senkung der Cholesterin- und Triglyzeridwerte im Sinne einer positiven Beeinflussung pathologischer Fettstoffwechselstörungen und somit Herabsetzung atherogener Risikofaktoren,
- die Senkung der alkalischen und sauren Phosphatase tierexperimentell wie klinisch im Sinne einer positiven Beeinflussung pathologischer Störungen von Knochen, Leber, Nieren, Darm oder von Leukämie oder Karzinom,
- fehlende Anzeichen mikromorphologischer Strukturstörungen oder pathologisch alterierter Organfunktionen im Sinne von unerwünschten - insbesondere cancerogenen bzw. mutagenen - Wirkungen aufgrund der Ergebnisse der zur Auswertung gelangten hämatologischen, klinisch-chemischen sowie der histologischen Untersuchungen.

Aus diesen experimentellen und klinischen Ergebnissen kann die Schlußfolgerung gezogen werden, daß die registrierten Änderungen definierter hämatologischer und klinisch-chemischer Analysenwerte sowie morphofunktioneller Zustandsbilder von Knochen und Knochenmark, von primären und sekundären lymphatischen Organen, sowie von Leber, Pankreas und Nieren unter zwei Aspekten zu betrachten sind: einerseits sprechen die vorliegenden Ergebnisse für Osteopathien-hemmende Effekte der Prüfsubstanz PO, andererseits für neue biologische Wirkungen, die unter therapeutischen Aspekten bei Defiziten des zellulären Immunstatus sowie bei Störungen des Knochenstoffwechsels (Osteoneogenese) und der Leber- und Nierenfunktion Verwendung finden können.

In einer Pilotstudie konnte nachgewiesen werden, daß in einer Kultur von humanen Osteoblasten diese nach Zusatz von PO zum Medium zu einer vermehrten Bildung von Kollagen Typ 1 sowie von Osteopontin und Osteocalcin angeregt werden konnten.

In einer Pilotstudie an Kaninchen wurde durch lokale Applikation extrakorporaler Stoßwellen auf das Dammbein eine umschriebene Neubildung von Knochengewebe in der Spongiosa induziert. Bei zusätzlicher oraler Applikation von PO wurden der Umfang der Neubildung erhöht und der zusätzliche Ablauf des Remodelling beschleunigt.

Bei der erfindungsgemäßen PO-Therapie stehen im Mittelpunkt:
- Verhinderung bestimmter spontaner Frakturen an typischer Stelle bei statischer Insuffizienz des Skelettes durch Verbesserung der Mikroarchitektur infolge der Induktion und Stimulation lokaler Mikrokallusbildung,
- Stimulation der Osteoneogenese und Hämatopoese
- Chemoprävention bei begleitender Therapie mit chemisch definierten Diuretika, Zytostatika und Kortikoiden (Hemmung der substanzspezifischen Osteoporose-Knochenmarkaplasie-Effekte und Verbesserung der Verträglichkeit einer begleitenden Hormonbehandlung,
- Minderung des oft frühzeitig einsetzenden Belastungsschmerzes der Wirbelsäule sowie der Gelenke.

Das Verfahren zur Herstellung von Putamen ovi (aus medizinischer Sicht ein Organtherapeutikum) insbesondere mit einer Komgröße von weniger als 0,1 mm gemäß WO 96/00578 wird vorzugsweise dadurch realisiert, daß man die Eierschalen von frischen Eiern (nicht aus längerfristiger Lagerung) aus kontrollierter Haltung nach EG- oder US-Recht verwendet.

Die Auswahl erfolgt nach individueller Durchleuchtung jedes Eies zur Prüfung des Qualitätsstatus (Unversehrtheit und Frischegrad).

Nach Aufschlag des Eies von Hand oder technische (Verwendung von Eiklar und Eigelb unverändert, gekühlt zur direkten Verwertung für Bäcker- und Konditorei-Betriebe (als zusätliches Qualitätskriterium), Sammeln der Eischalen und Tiefgefrieren (-25 °C), ist die Weiterverarbeitung innerhalb von 4 Tagen, nach umfassender analytischer Prüfung der Eischalenqualität durch visuelle und Geruchsprobe (frischer Eigeruch muß erhalten sein) geboten.

Die Eischalen, insbesondere von Gallus gallus domesticus werden bei Raumtemperatur oder erhöhter Temperatur mit gereinigtem Wasser unter Rühren gewaschen und die von Schmutzbestandteilen gereinigten Eischalen einer Autoklavierung unterworfen.

Die Autoklavierung erfolgt vorzugsweise diskontinuierlich; dabei werden in bestimmten Zeitabständen Luft und Wasserdampf vollständig entfernt und durch neubereiteten Wasserdampf ersetzt. Die Autoklavierung hat nicht allein die Aufgabe, Mikroorganismen zu inaktivieren und Schmutzbestandteile wie auch Lagerschutzmittel zu eliminieren, sondern ein Toxin- und Allergenpotential zu inaktivieren, das mit der Anwesenheit definierter - zum Teil auch denaturierter - Ovoproteine assoziiert ist. Durch den Einsatz und die relativ lange Einwirkungszeit des gespannten Wasserdampfes werden Membran- und Palisadenproteine und -glukoproteine gelartig gelöst und insbesondere durch die diskontinuierliche Arbeitsweise des Verfahrens weitgehend eliminiert.

Mittels der Zeitsteuerung werden 2 unterschiedliche Produkte erzeugt:
1. Autoklavierung bis ca. 3 Std.:
   Eischalenbestandteile der zentralen Palisadenzone, befreit von der inneren und äußeren Schalenmembran (PO)
2. Autoklävierung ca. 6 - 10 Std.:
   Eischalenbestandteile der zentralen Palisadenzone, befreit von der inneren und äußeren Schalenmembran sowie der Schalenmatrix der Palisadenzone (POM)

Während sowohl PO als POM oral appliziert osteoneogenetisch wirksam sind, ist POM auch geeignet als Ausgangsmaterial für die lokale Applikation als Knochenersatz.

Anschließend werden die Eischalen getrocknet und im Anschluß oder während der Trocknung auf die gewünschte Korngröße zerkleinert.

Die Wirkstoffe der Eischalen können wie folgt eingeteilt werden:

Die Eierschalenbestandteile POM können nicht nur mittels des Verfahrens gemäß WO 96/00578 hergestellt werden (POM₁) sondern auch mit Hilfe
- der enzymatischen Behandlung (POM₂)
   Ausgangsstoff is PO, welcher mit 1 - 10 % Lösung von Proteasen (proteolytische Enzyme), gepuffert, bei pH 7 bis 8, bei 30 bis 40 °C, über einen Zeitraum von 24 bis 72 Std. inkubiert und anschließend zur Reinigung erneut autoklaviert wird (1-3 Std.)
- der alkalischen Behandlung (POM3)
   mit siedender, wässriger 1-5 % NaOH-Lösung, anschließende Reinigung mittels Autoklavierung (1-3 Std.).

Die Schalenmatrix der Palisadenzone MPM mit organisch gebundenen Mineralstoffen kann auf drei Wegen isoliert werden:
- die Mineralstoff-haltigen Glukoproteine (MGP₁) werden mittels Autoklavierung gelartig gelöst; dabei wird MGP₁ aus der ausgetauschten wässrigen Phase mittels thermischer, Vakuum- und/oder Gefriertrocknung isoliert,
- die Mineralstoff-haltigen Glukopoteine (MGP₂) werden mittels wässriger und/oder organischer Lösungsmittel auch mitteis überkritischer Gase (CO₂-Extraktion) gelartig gelöst und durch anschließende thermische, Vakuumund/oder Gefriertrocknung isoliert,
- MGP₃ (denaturiert) fällt bei der Herstellung von POM₃ an. Nach alkalischer Behandlung von POM mit siedender 1-5 % NaOH-Lösung wird die Lösung von dem verbleibenden Schalenskelett abfiltriert und neutralisiert. Anschließend wird mittels Vakuum- und/oder Gefriertrocknung MGP₃ isoliert.

### Ausführungsbeispiet 1a bis c/ Vergleichsbeispiel 1a bis 1h:

### Tiere und Tierhaltung

Männliche Wistar-Ratten mit einem durchschnittlichen Körpergewicht von 322, 5 g wurden unter konventionellen Bedingungen bei einer Raumtemperatur von 21 +/-1°C, einer relativen Luftfeuchtigkeit von etwa 60 % und einem 12 Std.-Tag/Nacht-Rhythmus gehalten. Sie unterlagen vor Versuchsbeginn einer Akklimatisation von 12 Tagen an diese Haltungsbedingungen.

Die Ernährung erfolgte mit der pelletierten Standarddiät Altromin C1000, Misch.Nr. 100 (Firma Altrogge, Lage). Als Trinkwasser erhielten die Tiere Leitungswasser ad libidum.

### Prüfsubstanzen und Dosierung

### Beispiel 1a:

Drageekemgranulat mit Putamen ovi mikronisiert hergestellt durch Autoklavieren analog Beispiel 1 der WO 96/00578 mit einem Gewicht von 640 mg enthaltend Putamen ovi mikronisiert 440 mg, entsprechend 160 mg Calcium.

### Applikationsform:

Putamen ovi Drageekerngranulat 146 mg (≅ 100 mg Putamen ovi mikronisiert ≈ 36, 5 mg Ca) werden in 2 ml Aqua dest. suspendiert (POS), 0, 5 ml (POS) ≅ 9, 125 mg Ca (PO) / 250 mg KGW
Vergleichsbeispiel 1a:
   Calciumcarbonat (CC)
   91, 18 mg CC ≈ 36, 5 mg Ca
   0, 5 ml CCS ≈ 9, 125 mg Ca (CC) / 250 mg KGW
Vergleichsbeispiel 1b:
   Natriumfluorid (NF)
Vergleichsbeispiel 1c:
   Cyclophosphamid (CyS)
   Applikationsform: 1 mg Cyclophosphamid /0, 05 ml Aqua dest.
Vergleichsbeispiel 1d:
   Furosemid (FUR)
Beispiel 1b:
   Cyclophosphamid (CyS) + Putamen ovi (PO)
Vergleichsbeispiel 1e:
   Cyclophosphamid (CyS) + Calciumcarbonat (CC)
Vergleichsbeispiel 1f:
   Cyclophosphamid (CyS) + Natriumfluorid (NF)
Beispiel 1c:
   Furosemid (FUR) + Putamen ovi (PO)
Vergleichsbeispiel 1g:
   Furosemid (FUR) + Calciumcarbonat (CC)
Vergleichsbeispiel 1h:
   Furosemid (FUR) + Natriumfluorid (NF)

### Behandlungsgruppen und Applikation

31 männliche Wistar-Ratten wurden zur Untersuchung in folgende Behandlungsgruppen eingeteilt:

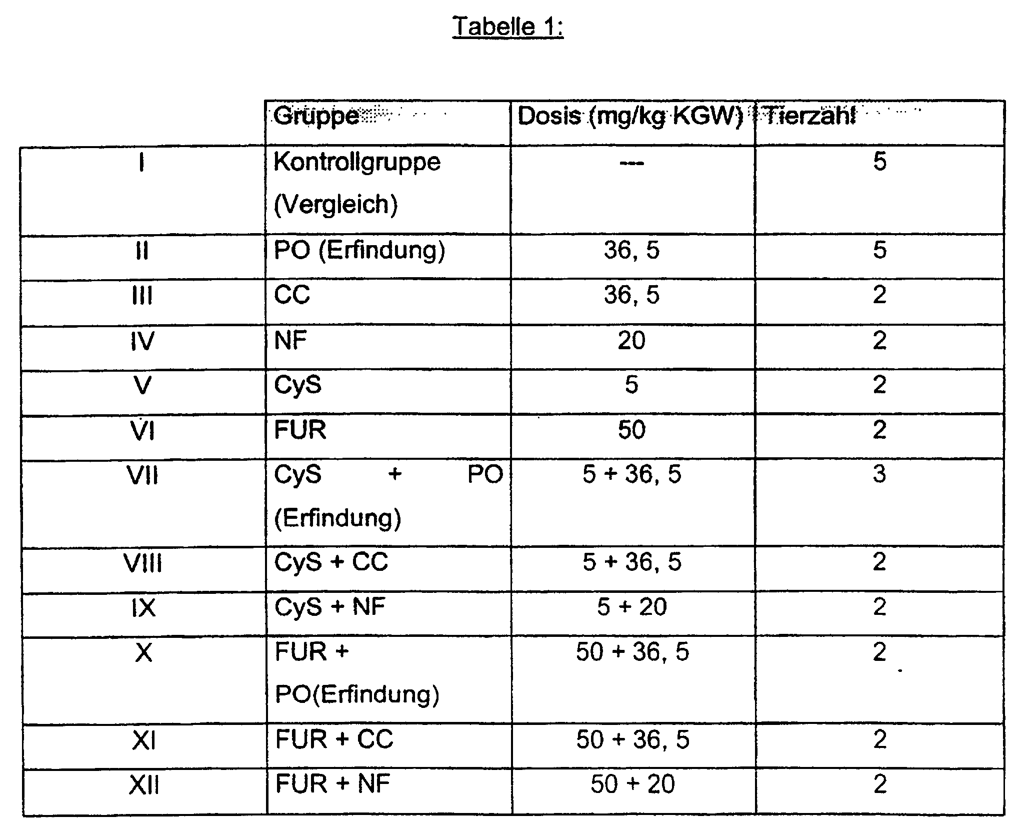

Die Prüfsubstanzen PO, CC, NF, CyS, FUR, CyS + PO, CyS + CC, Cys + NF sowie FUR + PO, FUR + CC und FUR + NF wurden einmal täglich 7 Tage lang den nicht sedierten Tieren intragastral mit Hilfe einer starren Knopfsonde verabreicht. Die Suspensionen wurden unmittelbar vor ihrer Applikation frisch hergestellt und homogen appliziert.

Die Kontrolltiere erhielten physiologische NaCl-Lösung in äquivalentem Volumen.

### Messung hämatologischer und klinisch-chemischer Parameter

Im einzelnen wurden im peripheren Blut erfaßt:
- kombiniert:
   Erythrozyten (RBC), Leukozyten (WBC), Thrombozyten (PLT), Hämoglobin (HGB), Hämatokrit (HCT)
   Erythrozytenindizes:
      Mittleres Zellvolumen (MCV), Mittleres korpuskuläres Hämoglobin (MCH),
      Mittlere Hämoglobinkonzentration der Erythrozyten (MCHC) und Erythrozytenverteilungsbreite (RDW)
- durchflußzytometrisch:
   Leukozytendifferenzierung: Granulozyten, Monozyten, Lymphozyten, Bund
   T-Zellen, Helfer- und Suppressorzellen
- kombiniert:
   GPT, GOT,
   Glukose,
   Cholesterin, Triglyzeride,
   Na, K, Cl, Ca,
   Kreatinin, Harnstoff, Gesamtprotein.

Mit Hilfe des vollautomatischen Hämatologie-Analysengerätes Sysmex K-1000 konnten bestimmt werden: WBC, RBC, PLT sowie HGB und HCT, ferner MCV, MCH und MCHC. Die Haupteinheit dieses Gerätes bestand im wesentlichen aus einem hydraulischen (HS) und einem elektronischen Systen (ES). Mit Hilfe des HS wurde angesaugt, pipettiert, verdünnt, gemischt und lysiert. Das ES analysierte und wandelte die Signale des HS um und übermittelte die Ergebnisse an den Drucker. Das Es überwachte mit Hilfe von Mikroprozessoren ebenso die Testabläufe, die Teststation und führte die Qualitätskontrolle durch.

Der Hämatokritwert gab den prozentualen Volumenanteil der Erythrozyten im Blut an. Hämoglobin, das in den Erythrozyten enthaltene Chromoprotein , war neben der Erythrozytenzahl und dem Hämatokritwert ein wichtiges Kriterium der Diagnostik von Anämien. Die Klassifizierung erfolgte durch die Erythrozytenindizes. Erythrozytengröße und Hämoglobingehalt wurden durch das Erythrozytenvolumen (MCV = mean corpuscular volume), den Hämoglobingehalt der Erythrozyten (MCH = mean corpuscular haemoglobin) und die mittlere korpusculäre Hämoglobinkonzentration (MCHC = mean corpuscular haemoglobin concentration) gekennzeichnet. Die Erythrozyten-Verteilungsbreite (RDW = red cell distribution width) ist ein Maß der Anisozytose.

Die Parameter wie Enzyme, Glukose, Lipide, Elektrolyte, Kreatinin, Harnstoff und Protein wurden mit Hilfe des Analysengerätes Cobas Mira selektiv, methodenorientiert, photometrisch oder ionenselektiv erfaßt. Der Zusatzreport lieferte analysenspezifisch Daten der Qualitätskontrolle und Statistik.

Die Leukozytendifferenzierung wurde mit Hilfe des Durchflußzytometers FACScan nach entsprechender Lysierung der Vollblutprobe durchgeführt (Streulichtmessung).

Die Lymphozytendifferenzierung erfolgte nach spezifischer monoklonaler Inkubation mittels fluoreszensaktivierter Zellsortierung (Fluoreszensmessung).

Quantitativ wurden am 7. Behandlungstag analysiert:
- Leukozyten (gesamt), Lymphozyten (gesamt),
- T-Lymphozyten (CD2+ / CD45 RA-),
- B-Lymphozyten (CD2- / CD45 RA+),
- Helfer-Lymphozyten (CD4- / CD8b+),
- NK-Zellen (CD8a+ / CD8b-).

Zur Phänotypisierung der Lymphozyten wurden diese mit folgenden Antikörpern der Fa. Pharmingen, San Diego (USA) inkubiert, an welche ein Fluorochrom gekoppelt ist:

Fluorescein Isothiocyanate (FITC) conjugated Mouse Anti-Rat CD2 Monodonal Antibody, -Phycoerythrin (R-PE) conj. Mouse Anti-Rat CD45RA OR A/B Monoclonal Antibody, Fluorescein Isothiocyanate (FITC) Mouse Anti-Rat CD4 Monoclonal Antibody, R-Phycoerythrin (R-PE) conj. Mouse Anti-Rat CD8 (ββ-Chain) Monoclonal Antibody, Fluorescein Isothiocyanate (FITC) conj. Mouse Anti-Rat CD8a Monoclona Antibody.

| | | |
|---|---|---|
| Ansatz: | a) CD2/CD45RA | = T- und B-Zellen |
| | b) CD4/CD8b | = T₄- und T₈-Zellen |
| | c) CD8a/CD8b | = NK-Zellen |

Je 5 µl Antikörper werden mit 50 µl Na-EDTA-Blut bei Zimmertemperatur im Dunkeln 20 Min. lang inkubiert. Die Suspension wird mit 2ml Lyses Reagenz der Fa. Becton-Dickinson geschüttelt und wie beschrieben 10 Min. lang inkubiert. 6 Min. lang wird anschließend bei 400 G zentrifugiert, der Überstand abgegossen. Das Sediment wird mit 3 ml Cell-Wash gewaschen und 6 Min. lang bei 400 G zentrifugiert. Das Sediment wird in 100 µl Cell-Wash aufgenommen. Die Suspension wird mit Hilfe des Durchflußzytometers analysiert.

### Klinische Beobachtungen:

Während der Akklimatisation vor Versuchsbeginn und im Verlauf der gesamten Versuchsdauer wurde das Allgemeinbefinden der Tiere kontrolliert. Zusätzlich erfolgte täglich eine Bestimmung des Körpergewichtes.

### Histologische Untersuchungen

Die histologischen Untersuchungen wurden nach Formalinfixierung der Organproben an Paraffinschnitten (Medim-Plast®) mit Hämatoxylin-Eosin-Färbung (H.-E.) durchgeführt.

Folgende ausgewählte Organe gelangten zur lichtmikroskopischen Untersuchung: Peyersche Plaques, Knochenmark (sternal), Thymus, Milz, Lymphknoten (mesenteriales Einzugsgebiet) aus dem Bereich des Abwehrsystems sowie die Parenchyme Leber, Pankreas und Nieren.

### Ergebnisse:

### Erythrozyten und Leukozyten

Die Potentiale der Prüfsubstanzen PO, CC, NF, CyS, FUR, CyS + PO, CyS + CC, Cys + NF sowie FUR + PO, FUR + CC und FUR + NF wurden indirekt durch ihre Stimulationswirkung auf die Anzahl der Leukozyten (WBC) und Erythrozyten (RBC) bestimmt.

Für folgende Prüfsubstanzen wurden gemessen (entsprechende Werte der Kontrollgruppe = 100 %):

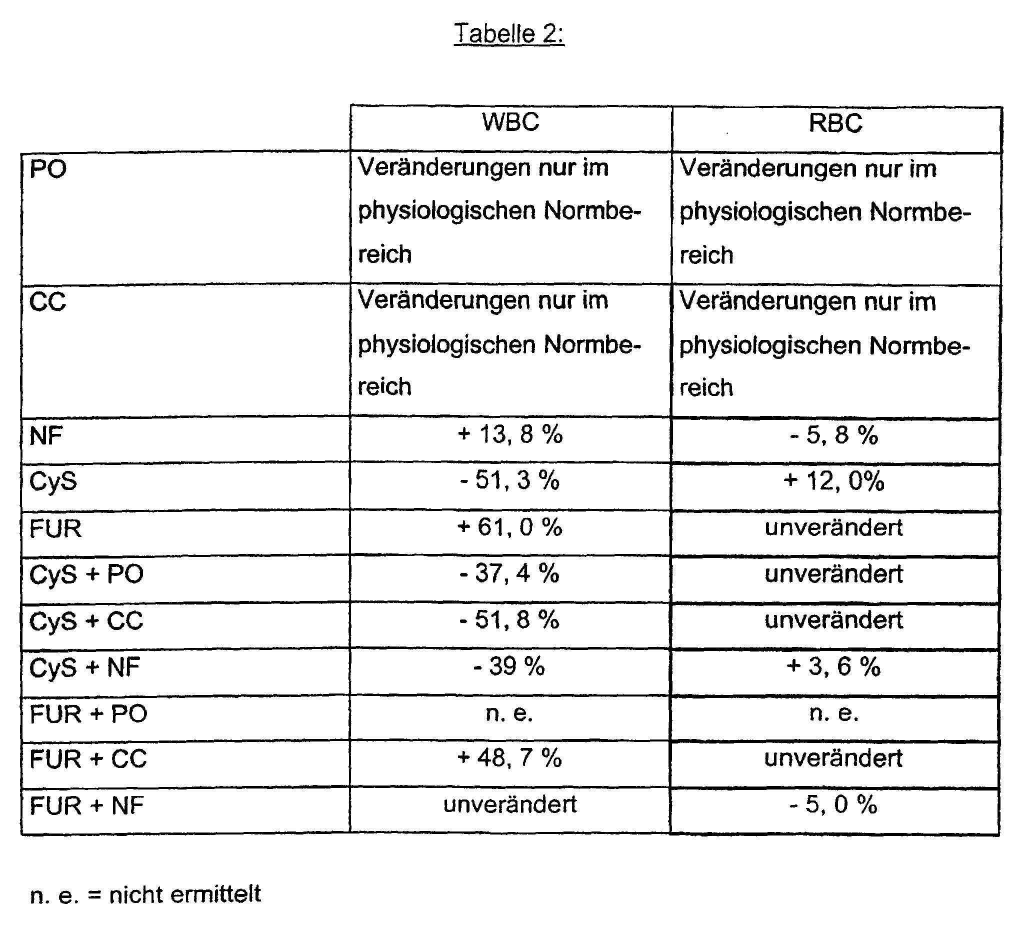

### Bilirubin, Kreatinin, Harnstoff, Glukose, Lipide, Elektrolyte und Eiweiß

Folgende klinisch-chemische Stoffwechsel-Meßgrößen wurden kombiniert methodenspezifisch analysiert:

Bilirubin, Kreatinin, Harnstoff, Glutamat -Oxalazetat-Transaminase (GOT), Glutamat-Pyruvat-Transaminase (GPT), Gamma-Glutamyltransferase (GGT), Glucose, Cholesterin, Triglyzeride, Kalzium, Natrium, Kalium, Chlorid und Gesamtprotein.

Für folgende Prüfsubstanzen wurde gemessen:

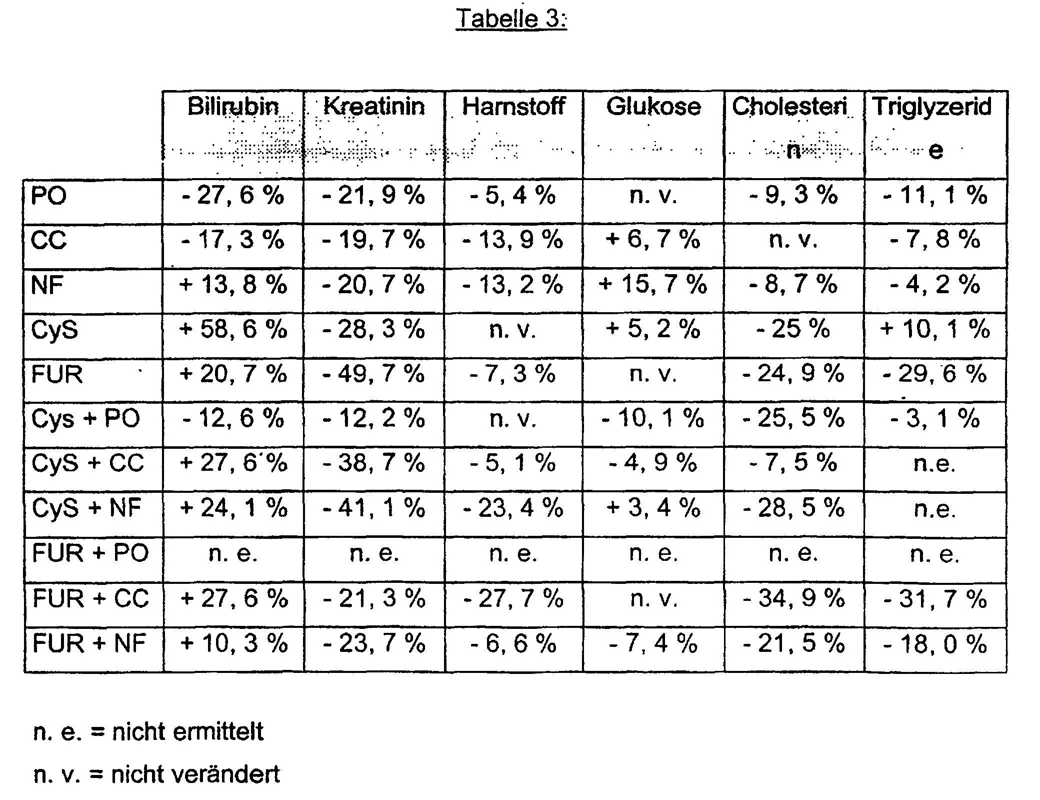

Für folgende Prüfsubstanzen wurde gemessen:

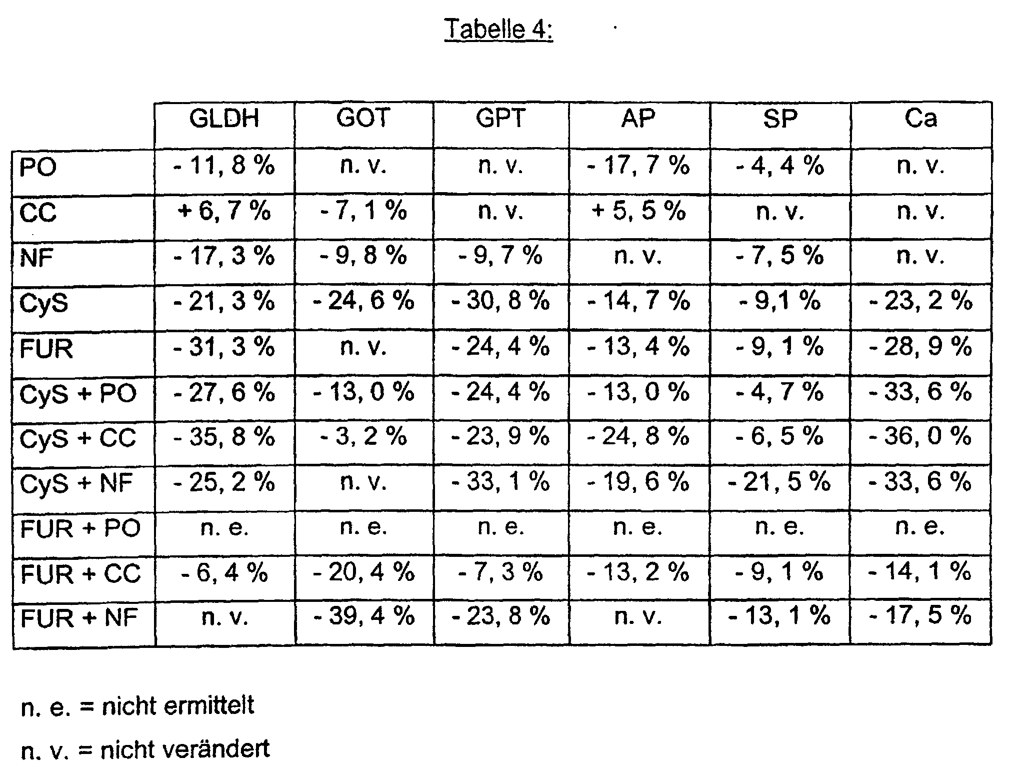

### Lymphozytendifferenzierung

Für folgende Prüfsubstanzen wurde gemessen:

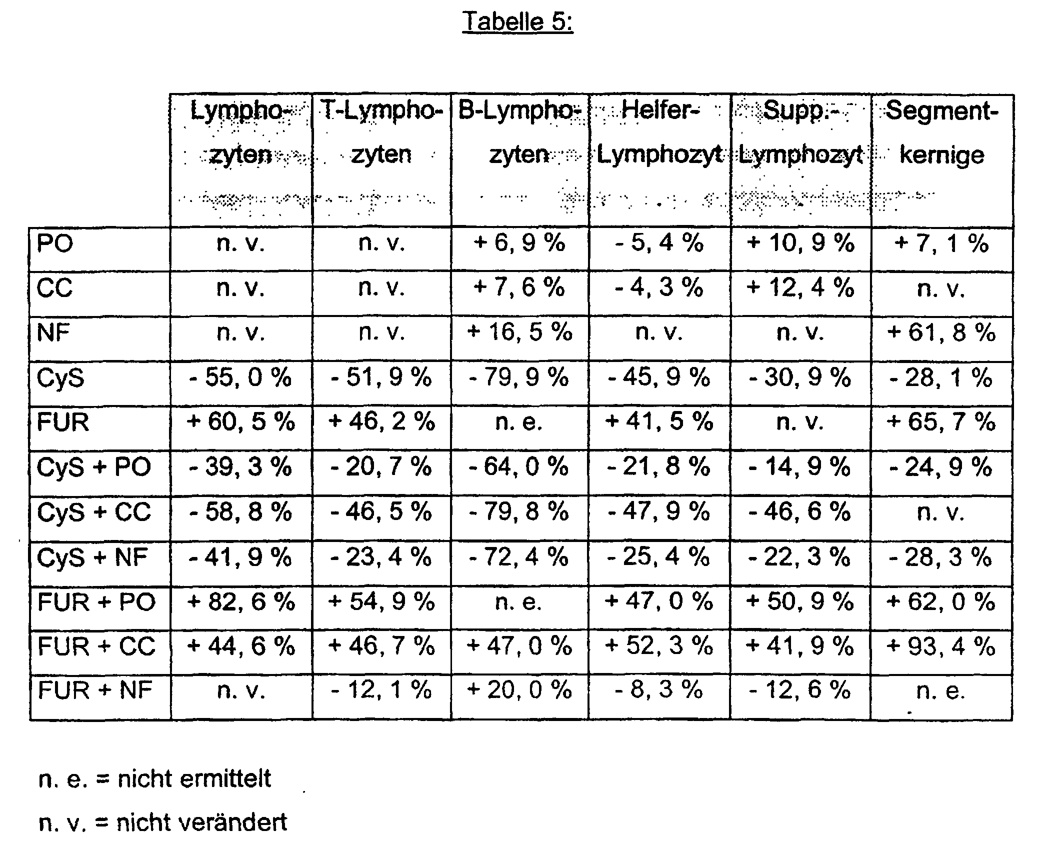

### Ausführungsbeispiel 2 (Referenzbeispiel):

Klinische Untersuchung zur Induktion der Osteoneogenese sowie zur Reduktion des Belastungsschmerzes des Bewegungsapparates

### Patientenstudie

Die klinischen Untersuchungen zur Wirkung von PO gemäß Beispiel 1 auf die Osteoneogenese bei 41 Patientinnen mit verringerter Knochendichte in der Postmenopause sowie auf die Verringerung des bestehenden Belastungsschmerzes (Schmerzreduktion) der Wirbelsäule und der Gelenke.

Die Untersuchungsergebnisse sind Resultate wiederholter osteodensitometrischer Bestimmungen.

### Osteodensitometrie

Die Knochenmineraldichte-Messungen wurden mittels der quantitativen digitalen Radiographie (Hologic QDR-1000 TM bone densitometer) an den Lendenwirbeln (LWK 1 - 4) durchgeführt, wobei das Ergebnis der Knochenmineraldichte-Berechnung aus Dichte in Gramm Calcium-Hydroxyapatit/cm ausgedrückt wird.

Bei diesem osteodensitometrischen Verfahren wird der Meßwert für die Absorption im Weichteilmantel eliminiert, so daß der Knochenmineralgehalt ohne Weichgewebefehler bestimmt werden kann.

### Erstuntersuchung der Lendenwirbelsäule (LWK 1 - 4) bei Patientinnen in der Postmenopause

Die Ergebnisse der densitometrischen Erstuntersuchung eines größeren Patientenkollektivs, mindestens ein halbes Jahr nach der letzten Menstruation, wurde auf der Basis von Referenz-Daten (fast 1000 Lendenwirbelsäulenmessungen) altersspezifisch verglichen. Die gemessene Knochendichte (g/cm³) wurde im Vergleich zum altersentsprechenden Referenzwert zusätzlich auch in Prozent angegeben. 41 Patientinnen in der Postmenopause, deren ermittelte Knochendichte zwischen 61 und 92 % lag, wurden anschließend in die Untersuchung aufgenommen.

### Vergleich von zwei Wirbelsäulenscans zur Verlaufskontrolle

Während der Therapiephase mit PO wurden keine zusätzlichen Hormon-, Vitamin D₃-, Calcitonin- sowie andere Calcium-Präparate appliziert.

Zur Verlaufskontrolle der therapeutischen Wirkung von PO bei den 41 ausgewählten Patientinnen mit verringerter Knochendichte in der Postmenopause wurde der jeweilige Meßwert nach Therapieende mit dem des Therapiebeginns verglichen. Hierzu war es unbedingt erforderlich, die Meßfelder der Wirbelsäulenscans hinsichtlich Größe, Form und Lage der beiden Analysen identisch zu justieren. Ermöglicht wurde dies durch die Anwendung der Hologic-spezifischen computergesteuerten Vergleichseinrichtung, bei der das Meßfeld der Wiederholungsuntersuchung mit demjenigen der Erstuntersuchung aufgrund des Abgleichs mit dem gespeicherten Scan optisch identisch eingerichtet wird. Damit wird die Reproduzierbarkeit der Meßgenauigkeit optimiert. So ist mit diesem Verfahren sichergestellt, daß die Abweichungen der Meßwerte zu unterschiedlichen Zeitpunkten dominant durch Änderungen im Knochenstoffwechsel zustande gekommen sein müssen.

### Ergebnisse

Die Gesamtbilanz der osteodensitometrischen Verlaufskontrollwerte auf der Basis der 41 Verlaufsuntersuchungen zur Wirkung von PO zeigt eine durchschnittliche Zunahme der Knochenmineraldichte um + 9, 4 % (von 78, 1 % auf 85, 5 %).

In der Gruppe A, die weniger als 200 Tage lang PO eingenommen hat, stieg die Knochendichte bereits durchschnittlich um 5, 5 %. In der Gruppe B, die weniger als 300 Tage PO eingenommen hat, stieg der Meßwert auf 7, 3 %, in der Gruppe C (länger als 300 Tage) stieg der Meßwert sogar auf 9, 4 %.

Die Zunahme der Meßwerte nach Therapieende, die in der Patientinnengruppe A (Knochendichte vor Therapiebeginn > 80 %) und Gruppe B (< 80 %) erhoben wurden, ist deutlich unterschiedlich. Die Ergebnisse zeigen eine deutliche Dosis-Wirkung-Beziehung: in der ersten Gruppe (A) stieg der Wert unter einer Applikation von 3 x 1 Dragee PO/Tag um 6, 9 %, in der zweiten Gruppe (B) bei 3 x 2 Dragees PO/Tag um 10, 9 %.

Der Wirkungsgrad der PO-Therapie bei verminderter Knochenmineraldichte in der Postmenopause (responder/non responder-Verhältnis) stellt sich wie folgt dar:

Bei 12 der untersuchten Patientinnen lag die Zunahme der Knochendichte unter 5%, bei 18 Patientinnen zwischen 5 und 10 %, bei 11 Patientinnen lag der Wert über 10% (+ 15, 4 %). Die Responderrate kann somit mit ca. 70 % angenommen werden (B + C).

Bei keiner Patientin lag nach Therapieende die Knochendichte unter derjenigen der Erstuntersuchung.

Durch die Therapie mit PO ist es offensichtlich möglich, allgemein einer Knochendichteminderung der Wirbelsäule bei Patientinnen in der Postmenopause entgegenzuwirken.

Die Untersuchung zeigte außerdem, daß das Maß der Zunahme der Knochenmineraldichte abhängig ist von der Höhe der Tagesdosis, wie auch der Dauer der PO-Therapie.

## Patentansprüche

1. Verwendung von Putamen ovi zur Herstellung von oral applizierbaren Arzneimitteln zur Behandlung oder Prävention von strahlen-, infektions- und chemisch bedingten Organ- und Gewebeschädigungen sowie zur zellulären Immunstimulation **dadurch bedingter** immunsupprimierter Zustände.

2. Verwendung gemäß Anspruch 1, wobei die Organ- und Gewebeschädigungen umfassen:
Schädigungen des O-MALT-Systems des Dünndarms, des Knochenmarks (Knochenmarkaplasie),
Knochen- und Knorpel-Genesestörungen,
Krankheiten des Bewegungsapparates,
Dysfunktion, Aplasie oder Hypoplasie des Thymus,
Dysfunktion der Milz und Aplasie oder Hypoplasie infolge medikamenten- oder strahlenbedingter Schädigung der Lymphknoten,
Atrophie, Nekrose der Leber,
Insuffizienz der exokrinen Sekretion von Proteasen, Esterasen, Carbohydrasen und Nukleasen der Bauchspeicheldrüse,
Insuffizienz der endokrinen Funktion der Langerhansschen Inseln, des Kohlenhydratstoffwechsels und der Nieren,
Leukozytopenie, Granulozytopenie, Lymphozytopenie, Thrombozytopenie, Erythrozytopenie,
Immunglobulinmangelzustände, auch infolge von AIDS und Tumoren sowie
Hyperlipoproteinämien und Hyperlipidämie.

3. Verwendung nach Anspruch 1 zur zellulären Immunstimulation, **dadurch gekennzeichnet, dass** sie insbesondere nach einer Immunsuppression nach Strahlen- oder Chemotherapeutikabehandlung die Erhöhung der Zellzahlen von Leukozyten, Thrombozyten, Erythrozyten, segmentkernigen Granulozyten und Lymphozyten, T-, B-, Helfer-, Suppressor- und NK-Zellen, insbesondere von B-Lymphozyten in der terminalen Strombahn betrifft, wobei eine Zunahme vitaler Lymphozyten in der Rindenzone und den -follikeln in den Peyerschen Plaques erkennbar ist, die mit einer sekretorischen Induktion von sIGA in den Dünndarm verbunden ist und/oder
eine zelluläre Stimulation Immunkompetenter Zellen in Thymus, Milz und mesenterialen Lymphknoten betrifft und/oder
die Aktivierung der Stoffwechselfunktionen von Leber, Pankreas und Nieren durch Senkung der Serumwerte von Kreatinin, Harnsäure, Glukose, Bilirubin, Cholesterin und Triglyzeriden sowie GOT, GPT und GGT betrifft,

4. Verwendung nach Anspruch 1 oder 2 zur Behandlung
von primären oder sekundären Störungen oder Schädigungen chondraler oder desmaler Ossifikation, der Osteoneogenese und Hämatogenese, gegebenenfalls in Kombination mit Natriumfluorid und Hormonen, insbesondere Östrogenen, Calcitonin, Pyrophosphate, insbesondere Biphosphonate und Vitaminen, insbesondere Vitamin D, insbesondere D3 und Dihydroxicolecalciferol,
der Knochensubstanz mit äußerer Substantia corticalis und innerer Substantia spongiosa,
des Skelettes inklusive Knochenzellen, Interzellulärsubstanz mit kollagenen Fibrillen und verkalkter Kittsubstanz,
des Knochenmetabolismus inklusive der Funktion von Osteoklasten und Osteoblasten und
der Abstimmung zwischen Knochenresorption und -formation.

5. Verwendung nach einem der Ansprüche 1 bis **4** zur
Stimulation der Stoffwechselleistung der Osteoblasten-spezifischen Transkriptionsfaktoren, der Differenzierung der Vorläuferzellen zu Osteoblasten durch Regulierung der Osteoblastendifferenzierung und
Stimulation der Bereitstellung der knochenspezifischen alkalischen Phosphatase, der kollagene, insbesondere Kollagen Typ I sowie von Osteopontin und Osteocalcin.

6. Verwendung nach einem der Ansprüche 1 bis **4** in der Kiefer- und Knochenchirurgie nach oraler und lokaler Applikation zur Stimulierung der Osteoneogenese und der frühzeitigen Kallusbildung.

7. Verwendung nach Anspruch 1 oder 2 in Kombination mit Zytostatika, insbesondere Cyclophosphamid oder Strahlentherapie als orale Adjuvanzien der malignen Tumorbehandlung, Insbesondere der Behandlung von primären und sekundären Knochentumoren mit osteoklastischen oder - lytischen Metastasen, tumorähnlichen Knochendefekten mit intraossärer Raumforderung, zur Behandlung von Knochenzysten.

8. Verwendung nach Anspruch 1 **oder 2** zur
Steigerung der Verträglichkeit von chemo-, zytostatischer, radiologischer Therapie, insbesondere in Kombination mit Zystostatika, insbesondere Cisplatin, Cyclophosphamid, Methotrexat, Fluorouracil Bleomycin und/oder Diuretika, insbesondere Acetazolamid, Hydrochlorothiazid, Chlorthalidon, Furosemid, Amilorid zur
Hemmung des Diurese- und Salurese-bedingten Knochensubstanzabbaues, als
Adjuvanz der Analgesie und
zur positiven pharmazeutischen Beeinflussung der Wechselwirkungen zwischen Endokrinum, Nerven- und Immunsystem.

9. Verwendung nach einem der Ansprüche 1 bis **5** zur Hemmung der ossären Entkalkung bei täglichem Zigarettenkonsum.

10. Verwendung nach Anspruch 1 **oder 2** zur additiven Behandlung von Hyperlipoproteinämien, insbesondere Hypercholesterinämie und/oder Hypertriglyzeridämie.

11. Verwendung nach einem der Ansprüche 1 **bis 3** zur Behandlung von Anämie insbesondere bei Tumorpatienten.

12. Verwendung nach einem der Ansprüche 1 bis **11** als Putamen ovi pulvis und in extrahierter Form mit Hilfe von wässrigen und/oder organischen und/oder überkritischen Lösungsmitteln, insbesondere Kohlendioxid; als Pulver, Granulat, Kapsel, Tablette, Dragee, Pastille oder Paste.

13. Verwendung nach Anspruch 12 zur oralen und lokalen Applikation auf der Basis von
- Eischalenbestandteilen der zentralen Palisadenzone, befreit von der inneren und äußeren Schalenmembran,
- Eischalenbestandteilen der zentralen Palisadenzone, befreit von der inneren und äußeren Schalenmembran sowie der Schalenmatrix der Pallsadenzone und/oder
- Schalenmatrix der Palisadenzone mit organisch gebundenen Mineralstoffen
zur Herstellung eines Mittels zur
Knochenremodellierung und Implantation, als Knochenzement und Knochenersatz mit osteoneogenese-aktivem Potential zur gezielten lokalen Knochenbildung,
zur Rekonstruktion von knöchernen Defiziten,
bei der Ausfüllung von knöchernen Schäden nach Tumoroperationen und zur Beseitigung von Knochenschäden in der Mund-, Kiefer- und Gesichtschirurgie und
lokalen Induktion einer Osteoneogenese durch Applikation extrakorporaler Stoßwellen im Kombination mit oraler Einnahme von Putamen ovi.

14. Verwendung nach einem der Ansprüche 1 bis **13, dadurch gekennzeichnet, dass** man Putamen ovi-Präparationen in Kombination mit Viscum-Extrakten, Echinacea-Extrakten, Cynara-Extrakten, Urtica-Extrakten, Betula-Extrakten, Equisetum-Extrakten und/oder Taraxacum-Extrakten, gegebenenfalls in Kombination mit Diuretika/Zytostatika zur Hemmung der Knochendemineralisation und/oder -depression einsetzt.

## Claims

1. Use of putamen ovi for the preparation of orally applicable medicaments for the treatment and prevention of organ and tissue damage caused by radiation, infections and chemically, and for cellular immunostimulation in immunosuppressed conditions caused by such damage.

2. The use according to claim 1, wherein said organ and tissue damage comprises:
damage to the O-MALT system of the small intestine, the bone marrow (bone marrow aplasia),
bone and cartilage genesis disorders,
diseases of the locomotor system,
dysfunction, aplasia or hypoplasia of the thymus,
dysfunction of the spleen and aplasia or hypoplasia due to damage to the lymph nodes caused by medicaments or radiation,
atrophy, necrosis of the liver,
insufficiency of the exocrine secretion of proteases, esterases, carbohydrases and nucleases of the pancrease,
insufficiency of the endocrine function of the islets of Langerhans, of the carbohydrate metabolism and of the kidneys,
leucocytopenia, granulocytopenia, lymphocytopenia, thrombocytopenia, erythrocytopenia,
immunoglobulin deficiency conditions, also due to AIDS and tumors, and hyperlipoproteinemias and hyperlipidemia.

3. The use according to claim 1 for cellular immunostimulation, **characterized by** relating to, especially following immunosuppression after radiation or chemotherapeutic treatment, the increase of the cell counts of leucocytes, thrombocytes, erythrocytes, polymorphonuclear granulocytes and lymphocytes, T, B, helper, suppressor and NK cells, especially of B lymphocytes, in the terminal vascular system, wherein an increase of vital lymphocytes is seen in the cortex zone and the follicles of Peyer's plaques which is accompanied by a secretory induction of sIGA in the small intestine, and/or relating to a cellular stimulation of immunocompetent cells in the thymus, spleen and mesenterial lymph nodes, and/or relating to the activation of the metabolic functions functions of liver, pancreas and kidneys by lowering the serum levels of creatinin, uric acid, glucose, bilirubin, cholesterol and triglycerides as well as GOT, GPT and GGT.

4. The use according to claim 1 or 2 for the treatment of primary or secondary disorders of or damage to chondral or desmal ossification as well as osteoneogenesis and hematogenesis, optionally in combination with sodium fluoride and hormones, especially estrogens, calcitonin, pyrophosphates, especially biphosphonates and vitamins, especially vitamin D, especially D₃ and dihydroxycholecalciferol, of the bony substance with external substantia corticalis and internal substantia spongiosa, of the skeleton including osteocytes, intercellular substance with collagenous fibrils and calcified cement, of bone metabolism including the function of osteoclasts and osteoblasts, and of the balance between bone absorption and formation.

5. The use according to any of claims 1 to 4 for the stimulation of the metabolic performance of osteoblast-specific transcription factors, of the differentiation of precursor cells to osteoblasts by regulation of osteoblast differentiation, and for the stimulation of the provision of bone-specific alkaline phosphatase, collagens, especially type I collagen, and of osteopontin and osteocalcin.

6. The use according to any of claims 1 to 4 in oral and bone surgery after oral and local application for the stimulation of osteoneogenesis and for early callus formation.

7. The use according to claim 1 or 2 in combination with cytostatic agents, especially cyclophosphamide, or radiation therapy as oral adjuvants of malignant tumor treatment, especially the treatment of primary and secondary bone tumors with osteoclastic or osteolytic metastases, tumor-like bone defects with intra-ossal space occupation, for the treatment of bone cysts.

8. The use according to claim 1 or 2 for increasing the tolerance for chemotherapy, cytostatic and radiologic therapies, especially in combination with cytostatic agents, such as cisplatin, cyclophosphamide, methotrexate, fluorouracil, bleomycin, and/or diuretics, such as acetazolamide, hydrochlorothiazide, chlorothalidone, furosemide, amiloride, for inhibiting the bone substance destruction due to diuresis and saluresis, as an adjuvant of analgesia, and for the positive influencing of interactions between the endocrine, nervous and immune systems.

9. The use according to any of claims 1 to 5 for the inhibition of bone decalcification upon daily cigarette consumption.

10. The use according to claim 1 or 2 for the additive treatment of hyperlipoproteinemias, especially hypercholesterolemia and/or hypertriglyceridemia.

11. The use according to any of claims 1 to 3 for the treatment of anemia, especially in tumor patients.

12. The use according to any of claims 1 to 11 in the form of putamen ovi pulvis and in a form extracted with aqueous and/or organic and/or supercritical solvents, especially carbon dioxide, as powder, granules, capsule, tablet, coated tablet, pastille or paste.

13. The use according to claim 12 for oral and local application on the basis of
- egg shell components of the central palisade zone, freed from inner and outer shell membranes;
- egg shell components of the central palisade zone, freed from inner and outer shell membranes and from the shell matrix of the palisade zone; and/or
- shell matrix of the palisade zone with organically bound minerals;
for the preparation of an agent for bone remodelling and implantation, for use as a bone cement and bone replacement with an osteoneogenesis-active potential for the well-aimed local bone formation, for the reconstruction of bony deficiencies, in the filling of bone damage after tumor operations, and for the removal of bone damage in oral surgery and plastic surgery of the face, and for the local induction of osteoneogenesis by the application of extracorporal shock waves in combination with oral administration of putamen ovi.

14. The use according to any of claims 1 to 13, **characterized in that** putamen ovi preparations are employed together with *Viscum* extracts, *Echinacea* extracts, *Cynara* extracts, *Urtica* extracts, *Betula* extracts, *Equisetum* extracts and/or *Taraxacum* extracts, optionally in combination with diuretics/cytostatic agents, for the inhibition of bone demineralization and/or depression.

## Revendications

1. Utilisation de *Putamen ovi* pour la préparation de médicaments administrables par voie orale pour le traitement ou la prévention de dommages causés à des organes et des tissus, par des rayonnements, des infections et un agent chimique, ainsi que pour l'immunostimulation cellulaire d'états d'immunosuppression causés par ceux-ci.

2. Utilisation selon la revendication 1, pour laquelle les dommages causés à des organes et des tissus comprennent :
les dommages du système O-MALT de l'intestin grêle, de la moelle osseuse (aplasie médulaire),
des troubles de la genèse des os et du cartilage,
des maladies de l'appareil moteur,
des dysfonctionnements, l'aplasie ou l'hypoplasie du thymus,
des dysfonctionnements de la rate et l'aplasie ou l'hypoplasie suite à des dommages causés par des médicaments ou des rayons, à des ganglions lymphatiques, l'atrophie, la nécrose du foie,
une insuffisance de la sécrétion exocrine de protéases, d'estérases, de carbohydrases et de nucléases du pancréas, une insuffisance de la fonction endocrine des îlots de Langerhans, du métabolisme des hydrates de carbone et des reins,
la l'eucocytopénie, la granulocytopénie, la lymphocytopénie, la thrombocytopénie, l'érythrocytopénie,
des états de déficience en immunoglobulines, également suite au SIDA ou à des tumeurs, ainsi que
l'hyperlipoprotéinémie et l'hyperlipidémie.

3. Utilisation selon la revendication 1, pour l'immunostimulation cellulaire, **caractérisée en ce qu'**elle concerne en particulier, après une immunosuppression par traitement thérapeutique par rayons ou chimique, l'augmentation du nombre de cellules de leucocytes, thrombocytes, érythrocytes, granulocytes à noyau segmenté et lymphocytes, cellules T, B, helper, suppresseur et NK, en particulier des lymphocytes B dans la partie terminale, une augmentation des lymphocytes vitaux étant visible dans la zone du cortex et les follicules du cortex, dans les plaques de Peyers, laquelle est liée à une induction sécrétoire d'IGA dans l'intestin grêle, et/ou concerne une stimulation cellulaire de cellules immunocompétentes dans le thymus, la rate et les glanglions lymphatiques mésentériques, et/ou
concerne l'activation des fonctions métaboliques du foie, du pancréas et des reins par diminution des valeurs sériques en créatinine, acide urique, glucose, bilirubine, cholestérol et triglycérides, ainsi que du GOT, du GPT et du GGT.

4. Utilisation selon la revendication 1 ou 2, pour le traitement de troubles ou dommages primaires ou secondaires de l'ossification enchondrale ou directe, de l'ostéonéogenès et de l'hématogenèse le cas échéant en association avec du fluorure de sodium et des hormones, en particulier des oestrogènes, de la calcitonine, des pyrophosphates, en particulier du biphosphonate, et des vitamines, en particulier la vitamine D, en particulier D3, et le dihydroxycolécalciférol, de la substance osseuse avec substance corticale externe et substances spongieuse externe,
du squelette, y compris des cellules osseuses, de la substance intracellulaire avec fibrilles de collagène et substance fondamentale calcifiée,
du métabolisme osseux, y compris de la fonction des ostéoclastes et des ostéoblastes, et de la coordination entre résorption et formation osseuse.

5. Utilisation selon l'une quelconque des revendications 1 à 4, pour la stimulation du métabolisme des facteurs de transcription spécifiques des ostéoblastes, de la différentiation des cellules précurseurs des ostéoblastes par régulation de la différentiation des ostéoblastes et de la stimulation de la préparation de la phosphatase alcaline spécifique des os, du collagène en particulier du collagène de type I, ainsi que de l'ostéopontin et de l'ostéocalcine.

6. Utilisation selon l'une quelconque des revendications 1 à 4, en chirurgie maxillaire et des os après administration orale et locale pour la stimulation de l'ostéonéogenèse et de la formation précoce de cal.

7. Utilisation selon la revendication 1 ou 2, en combinaison avec des cytostatiques, en particulier le cyclophosphamide ou une thérapie par irradiation, comme adjuvant oral du traitement de tumeurs malignes, en particulier le traitement de tumeurs primaires et secondaires des os avec métastases ostéoclastiques ou -lytiques, des défauts des os de type tumeur avec exigence spatiale intra-osseuse, pour le traitement des kystes osseux.

8. Utilisation selon la revendication 1 ou 2, pour augmenter la tolérance vis-à-vis d'une chimiothérapie ou d'une thérapie cytostatique ou radiologique, en particulier en combinaison avec des cytostatiques, en particulier le cisplatine, le cyclophosphamide, le méthotrexate, le fluorouracile, la bléomycine et/ou des diurétiques, en particulier l'acétazolamide, l'hydrochlorothiazide, la chlorthalidone, le furosémide, l'amiloride, pour inhiber la dégradation des substances osseuses causée par la diurèse et la saliurèse, comme adjuvant de l'analgésie et pour influencer positivement pharmaceutiquement les interactions entre les systèmes endocriniens, nerveux et immuns.

9. Utilisation selon l'une quelconque des revendications 1 à 5, pour inhiber la décalcification osseuse lors d'une consommation quotidienne de cigarettes.

10. Utilisation selon la revendication 1 ou 2, pour le traitement additionnelle de l'hyperlipoprotéinémie, en particulier de l'hypercholestérolémie et/ou de l'hypertriglycéridémie.

11. Utilisation selon l'une quelconque des revendications 1 à 3, pour traiter l'anémie, en particulier chez les patients porteurs de tumeurs.

12. Utilisation selon l'une quelconque des revendications 1 à 11, de poudre de *Putamen ovi* et d'un extrait obtenu à l'aide de solvants aqueux et/ou organiques et/ou supercritiques, en particulier le dioxyde de carbone ; sous forme de poudre, granulé, capsule, comprimé, dragée, pastille ou pâte.

13. Utilisation selon la revendication 12, pour l'administration orale ou locale, sur la base de:
- de constituants d'enveloppe d'oeuf de la zone de palissade centrale, exempts des membranes interne et externe de l'enveloppe ;
- de constituants d'enveloppe d'oeuf de la zone de palissade centrale, exempts des membranes interne et externe de l'enveloppe ainsi que de la matrice d'enveloppe de la zone de palissade, et/ou
- de la matrice d'enveloppe de la zone de palissade avec des matières minérales liées de manière organique, pour la préparation d'un agent pour le remodelage et l'implantation d'os, comme ciment osseux et substitut osseux avec potentiel actif ostéonéogénique pour la formation locale ciblée d'os, pour la reconstruction de déficits osseux,
lors du remplissage de dommages osseux après des opérations de tumeurs et pour la suppression de dommages osseux en chirurgie de la bouche, maxillaire et du visage, et pour l'induction locale d'une ostéonéogenèse par application d'ondes de choc extracorporelles en combinaison avec la prise orale de *Putamen ovi*.

14. Utilisation selon l'une quelconque des revendications 1 à 13, **caractérisée en ce que** l'on met en oeuvre des préparations de *Putamen ovi*, en combinaison avec des extraits de viscum, des extraits d'échinacea, des extraits de cynara, des extraits d'urtica, des extraits de betula, des extraits d'equisetum, et/ou des extraits de taraxacum, le cas échéant en combinaison avec des diurétiques/cytostatiques pour l'inhibition de la déminéralisation et/ou la dépression des os.
